# EUROPEAN PATENT SPECIFICATION

(11) **EP 0 794 249 B1**
(45) Date of publication and mention of the grant of the patent: **17.12.2003**
(21) Application number: 97104216.3
(22) Date of filing: 17.10.1995
(51) Int. Cl.: C12N 9/16, C12N 9/96, C12Q 1/42

(54) **Method for stabilizing an aqueous alkaline phosphatase solution**
Verfahren zur Stabilisierung von einer wässerigen Alkalische-Phosphatase Lösung
Méthode pour la stabilisation d'une solution aqueuse de phosphatase alkaline

(30) Priority: 21.10.1994 JP 25640494
(43) Date of publication of application: 10.09.1997
(62) Divisional of application: 95116338.5
(73) Proprietor: Daiichi Pure Chemicals Co., Ltd., Tokyo (JP)
(72) Inventor: Xu, Jingzhi, c/o Daiichi Pure Chem. Co., Ltd., Tokyo (JP); Nozawa, Masayuki, c/o Daiichi Pure Chem. Co., Ltd., Tokyo (JP)
(74) Representative: Albrecht, Thomas, Dr.

(56) References cited:
- EP-A- 0 042 969
- EP-A- 0 151 320
- EP-A- 0 269 979
- PORTMANN, PLATO ET AL: "Calf intestinal alkaline phosphatase. I. Improved isolation method and molar composition of the purified phosphatase" HELV. CHIM. ACTA (1982), 65(8), 2668-81 CODEN: HCACAV;ISSN: 0018-019X, XP000564563

## Description

### BACKGROUND OF THE INVENTION

### Field of the Invention:

The present invention relates to a method for stabilizing an aqueous solution of alkaline phosphatase or its derivatives used as a research reagent or in clinical diagnostics based on enzyme immunoassay.

### Description of Related Art:

Recently, enzyme immunoassay and chemiluminescence enzyme immunoassay are frequently used in clinical diagnostics Among the enzymes used in such immunoassays, alkaline phosphatase and its derivatives (hereinafter referred to as "ALP and/or related substances") are particularly important enzymes. Therefore, the stability of storage is essential for the performance of the resulting reagents or products to be assured.

It is a conventional practice that these ALP and/or related substances are stored and reacted in buffers such as HEPES (4-(2-hydroxyethyl)-1-piperazineethanesulfonic acid)(pH 7.5-8.2), TEA (triethanolamine)(pH 7.6-8.0), or Tris (Tris(hydroxymethyl)aminomethane)(pH 8.0-8.5). However, it has been noted that the stability of ALP and/or related substances in such buffer systems is not sufficient if the temperature is high (25°C or higher) or if the storage is for a long period.

Thus, although it is desired to store ALP and/or related substances in a solution state and react them at a temperature as high as 37°C to facilitate handling and obtain good reproducibility, the conditions under which they are used are in fact strictly limited. That is, they must be handled at low temperatures or stored in a freeze-dried state, which causes cumbersome handling and a rise in costs.

In an attempt to solve these problems, improvements have been made over many years in the technologies for stabilizing ALP and/or related substances in a solution state. Typical methods include chemical modification of enzymes and addition of divalent metal ions such as Mg²⁺, Zn²⁺, Mn²⁺, Cd²⁺, or water-soluble sulfides. However, no conventional methods have achieved satisfactory stabilization, leaving the unanswered problem of stability.

Portmann et al. (Helv. Chim. Acta, Vol. 65, 2668-2681 (1982)) have described a modified and improved method for isolation of calf intestinal alkaline phosphatase. According to this method, the phosphatase is obtained as a homogenous glycoprotein, containing firmly bound zinc, magnesium and phosphoric acid.

In view of the foregoing, the present inventors carried out careful studies of a method for stabilizing an aqueous solution of ALP and/or related substances, and found that these substances can be stored stably for a prolonged period at high temperatures when a compound having a binding activity reversible with respect to ALP is added to an aqueous solution of ALP and/or related substances. The present invention was accomplished based on this finding.

### SUMMARY OF THE INVENTION

Accordingly, an object of the present invention is to provide a method for stably storing an aqueous solution of ALP and/or related substances over a long period without deactivating these substances even at relatively high temperatures (25°C or higher temperatures).

According to the present invention, there is provided an enzyme solution comprising alkaline phosphatase or a derivative thereof and a compound having a binding activity which is reversible with respect to alkaline phosphatase, wherein said compound is selected from the group consisting of a phosphoric acid ester, an alkali metal salt of phosphoric acid or a phosphoric acid ester, and a mixture of them.

The above and other objects, features, and advantages of the present invention will become apparent from the following description.

### BRIEF DESCRIPTION OF THE DRAWINGS

Fig. 1 is a graph of the results of Example 6, showing the effect of stabilizing agents on an ALP-AFP compound.
Fig. 2 is a graph of the results of Example 7, showing the effect of stabilizing agents and heat on an ALP-AFP compound.

### DESCRIPTION OF PREFERRED EMBODIMENTS

In the present invention, the compounds which have a binding activity reversible with respect to ALP and which are used as the stabilizing agent are phosphoric acid esters, and their alkali metal salts or alkali metal salts of phosphoric acid. These can be used, in spite that they have not previously been used as they adversely affect the measurement system. Specific examples of the compound which may be mentioned are inositol phosphate and its salts, riboflavin phosphate and its salts, and serine phosphate and its salts. Examples of the alkali metal salts of phosphoric acid include potassium phosphates such as KH₂PO₄/K₂HPO₄. Examples of the inositol phosphate and its salts include sodium phytate (sodium inositol hexaphosphate).

These compounds may themselves be used as buffers or they may be used after they are added in another buffer. In any case, effects at the same level may be obtained.

The amount of these stabilizing agents which are used in the present invention varies depending on the stabilizing agent used, intended stabilizing effect, and the solubility. In general, they are preferably added in such an amount that makes their concentration in the aqueous solution containing ALP and/or related substances not less than 1 mM, and particularly 2.5-500 mM, in order to obtain a sufficient stabilizing effect. For example, in the case where an alkali metal salt of phosphoric acid is used, it is preferably used in such an amount that makes its concentration in the aqueous solution to fall in the range from 10 to 250 mM. If sodium phytate is used, concentrations lower than cases where other compounds are used, for example, a concentration as low as 1 mM, can produce a stabilizing effect because it has 6 phosphoric groups in one molecule.

The pH of the aqueous solution containing the stabilizing agent of the present invention is preferably in the range from 5.5 to 8.5, and particularly from 5.5 to 7.5. In this connection, it should be noted that even when the pH is adjusted to fall in the range from 5.5 to 8.5, stabilizing effects as obtainable in the present invention would not be attained if the stabilizing agent according to the present invention is not used.

The stabilizing method of the present invention can be applied to aqueous solutions containing ALP and/or derivatives of ALP. The derivatives of ALP are not particularly limited. Nonlimiting examples of the derivatives of ALP which may be mentioned are ALPs bound to proteins (antigens, antibodies, avidin, etc.), peptides, haptens, coenzymes (biotin, etc.), sugars (lectin, etc.), or to nucleic acids (DNA-probes, RNA-probes, etc.) The antigens and antibodies which may be used are not particularly limited, and their species, concentrations, and the like can be determined in accordance with the substance assayed, etc.

The concentration of the ALP and/or related substances in the aqueous solution is not particularly limited. It is preferred that the concentration be in the range from 0.05 to 5000 µg/ml, and in particular from 0.5 to 1000 µg/ml.

According to the stabilizing method of the present invention, ALP and/or related substances can be stored for a long period in the state of solution which is ready-to-use. The solution itself can be used in reactions. Also, since ALP and/or related substances are made stable at high temperatures, immunological reactions are accelerated to shorten the time required for the assay and to improve the sensitivity of assay. Moreover, ALP and/or related substances in reagents for clinical assay which are used in enzyme immunoassay, etc. do not suffer from a reduction in activity, and therefore, good assay reproducibility can be obtained.

### Examples:

The present invention will further be described by way of examples, which should not be construed as limiting the invention.

### Example 1:

### Stabilizing effects of various stabilizing agents on ALP:

### 1. Preparation of an ALP solution

### (1) Preparation of buffers

In 100 ml each of 2 different buffers (KH₂PO₄/K₂HPO₄, HEPES/sodium phytate) containing a stabilizing agent and 2 different conventional buffers (HEPES, TEA), the concentration being 100 mM throughout the four buffers, the following were added: sodium chloride (150 mM), BSA (1%), magnesium chloride (2 mM), zinc chloride (0.2 mM), and sodium azide (0.1%). Subsequently, 6N HCl was used to adjust the pH as follows:
KH₂PO₄/K₂HPO₄ = 7.0,
HEPES/sodium phytate=7.0
HEPES=7.0, and
TEA=7.6.

### (2) Preparation of ALP solutions

An ALP solution having a known concentration was diluted with each buffer obtained in step (1) above to make its concentration to 10 µg/ml.

### 2. Assay method

From each of the four ALP solutions (concentration: 10 µg/ml) described above, some in a predetermined amount was taken and subjected to a heat load test (50°C/10 min., stress (+)). At the same time, the remainder solution was stored at 4°C for 10 minutes (control, stress (-)). After completion of the load test, buffers were replaced with a TEA buffer using a PD-10 column (product of Pharmacia). The control groups also underwent this buffer exchange. From each of the samples in which buffers were replaced, 500 µl of solution was taken. 50 µl of p-nitrophenyl-phosphate was added. After the reaction was allowed to proceed for 2 minutes, a stop solution was added. The enzymatic activities before and after the load test were measured as absorptions at 405 nm.

The results are shown in Table 1a.

**Table 1a**

| Buffers | ALP (405 nm O.D.) | | |
|---|---|---|---|
| | Stress(-) | Stress(+) | Activity(%) {Stress(+)/(-)} |
| KH₂PO₄/K₂HPO₄ (pH 7.0) | 1.853 | 1.814 | 97.9 |
| | | | |
| HEPES/phytate (pH 7.0) | 1.625 | 1.571 | 96.6 |
| | | | |
| HEPES (pH 7.0) | 1.671 | 1.362 | 81.5 |
| | | | |
| TEA (pH 7.6) | 1.232 | 0.92 | 74.9 |

As is apparent from Table 1a, according to the method of the present invention, not less than 96% of the activity of ALP was retained even after the heat load test. In contrast, according to conventional methods, the activity was reduced to less than 82%.

### Example 2:

### Stabilizing effects of various stabilizing agents on ALP-labelled antibody of anti α-fetoprotein (AFP) (ALP-AFP)

### A. Preparation of an ALP-AFP compound:

### 1. Introduction of a thiol into an anti-AFP antibody using N-acetyl-DL-homocysteine thiolactone (AHTL):

(1) 0.6 mg/0.01 ml of AHTL was added to 5 mg of a purified α-AFT antibody in 0.5 ml of a buffer formed of 0.1 M NaHCO₃/0.15 M NaCl/0.2 M imidazol (pH 9.0) while stirring at 4°C. The reaction was continued for 1 hour.
(2) After completion of the reaction, the antibody sample was placed on a Sephadex G-50 column, and desalted with PBS/1.0 mM EDTA. The thiol-introduced antibodies transferred to a blank column were pooled and the amount of protein was determined. Excessive AHTL was eluted in the second peak.

### 2. Introduction of maleimide (MI) into ALP using sulfo-SMCC

(1) To 14.4 mg (102.5 nmol) of ALP (0.1 M Tris/0.15 M NaCl), 10 mg/ml of a sulfo-SMCC solution was added dropwise while stirring. The sulfo-SMCC solution was 1.5 mg (about 30 nmol) excessive with respect to the ALP. The reaction was allowed to proceed for 1 hour at room temperature.
(2) After completion of the reaction, the sample was placed on a Sephadex G-50 column, and desalted with PBS/1.0 mM EDTA buffer. The maleimide-introduced ALP (ALP-MI) transferred to a blank column was pooled and the amount of protein was determined. Excessive sulfo-SMCC was eluted in the second peak.

### 3. Reaction of the antibody and enzyme

The thiol-introduced antibody solution (364 µg/ml) was stirred, where an ALP-MI solution (306 µg/ml) was added dropwise. The reaction was allowed to proceed while stirring at room temperature for 2 hours.

### 4. Blockade of active groups with β-mercaptoethanol (β-ME)

To 100 volumes of the solution of the antibody-enzyme compound, 1 volume of β-ME was added so as to obtain a final concentration of 1 mM, and the mixture was incubated at room temperature for 30 minutes.

### 5. Separation of free antibodies and free enzymes from the antibody-enzyme compound by gel filtration

The solution of the antibody-enzyme compound was placed on a Gel SW-3000 column (product of Tosoh). Using 0.1 M Tris/0.15 M NaCl (pH 7.0) as a buffer for elution, the solution was fractionated until free antibodies and free ALP were eluted.

### B. Preparation of a solution of the ALP-AFP compound

### 1. Preparation of a buffer

Four different buffers shown in Table 1a were prepared in a manner similar to that described in step 1(1) of Example 1.

### 2. The ALP-AFP compound at a high concentration prepared in A above was diluted to a concentration of 1 µg/ml using the four buffers.

### C. Assay method

Some amount of each of the four ALP-AFP compound solutions was taken, and subjected to a heat load test (50°C, 10 minutes). The remainder solution was stored at 4°C for 10 minutes (control). When 10 minutes were passed, 50 µl of the solution was taken from each sample and combined with 50 µl of a solid phase bound to anti-AFP antibodies, 10 µl of an AFP antigen sample, and 50 µl of a liquid for dilution. The reaction was allowed to proceed for 5 minutes. Subsequently, the reaction was washed three times. Amadatylmethoxyphenyl phosphoryl dioxotane (AMPPD), which is the substrate for ALP, was added, and activities of the ALP-AFP compound before and after the loading test were measured.

The results are shown in Table 1b.

**Table 1b**

| Buffers | ALP-antibody compounds (Relative luminance unit: RLU) | | |
|---|---|---|---|
| | Stress(-) | Stress(+) | Activity(%) {Stress(+)/(-)} |
| KH₂PO₄/K₂HPO₄ (pH 7.0) | 45,689 | 43,823 | 95.9 |
| | | | |
| HEPES/phytate (pH 7.0) | 44,256 | 41,741 | 94.3 |
| | | | |
| HEPES (pH 7.0) | 46,119 | 33,943 | 73.6 |
| | | | |
| TEA (pH 7.6) | 41,335 | 30,629 | 74.1 |

According to the method of the present invention, not less than 94% of the activity of ALP-AFP compound was retained even after the heat load test. In contrast, according to conventional methods, the activity was reduced to less than 75%.

### Example 3:

### Stability test on ALP when the pH of buffers containing a stabilizing agent was changed:

### 1. Preparation of buffers

In 100 ml each of 5 different buffers including two inventive stabilizing buffers (K₂HPO₄/KH₂PO₄, HEPES/sodium phytate) and 3 different conventional buffers (MES, HEPES, TEA), the concentration being 100 mM throughout the five buffers, the following were added: sodium chloride (150 mM), BSA (1%), magnesium chloride (2 mM), zinc chloride (0.2 mM), and sodium azide (0.1%). Subsequently, 6N HCl was used to adjust the pH as follows:
K₂HPO₄/KH₂PO₄: 5.5, 6.0, 6.5, 7.0, 7.5, 8.0, 8.5
HEPES/sodium phytate: 7.0, 7.5, 8.0
MES: 5.5, 6.0, 6.5
HEPES: 7.0, 7.5, 8.0, 8.5, and
TEA: 7.6.

### 2. Assay method

Using the above-described buffers, 10 µg/ml of enzymatic solutions were prepared in a manner similar to that described in step 1(2) in Example 1. Subsequently, a heat load test described under item 2 in Example 1 was carried out. Enzymatic activities were measured.

The results are shown in Tables 2 and 3.

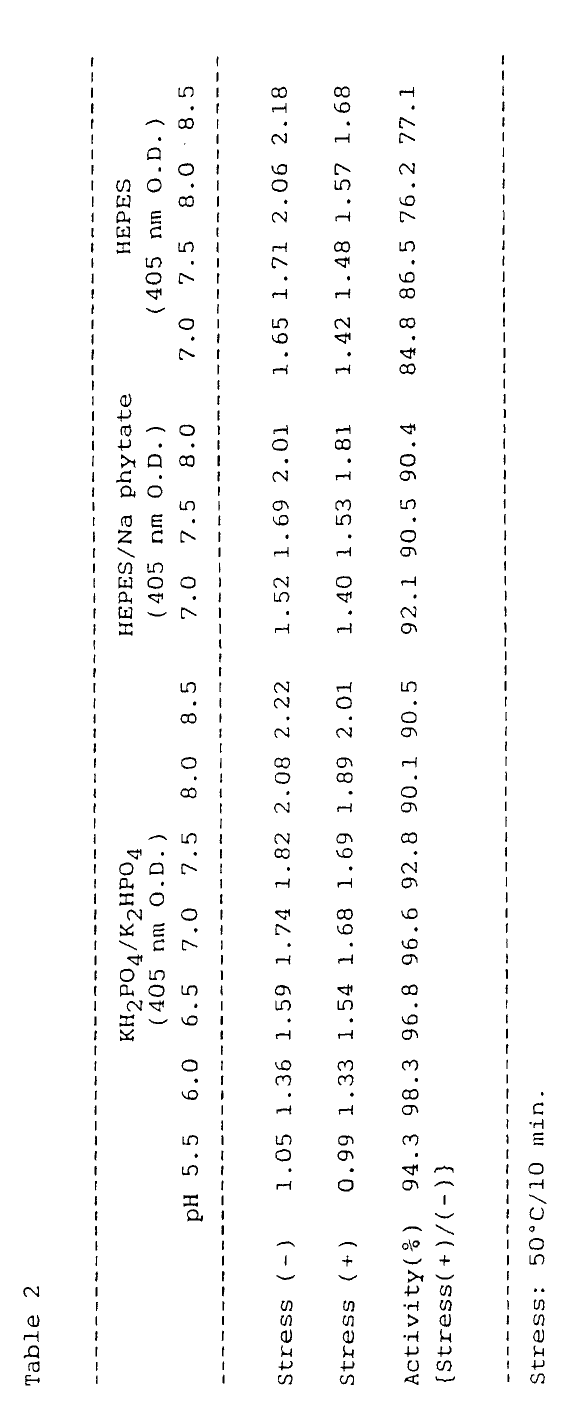

**Table 3**

| | MES (405 nm O.D.) | | | TEA (405 nm O.D.) |
|---|---|---|---|---|
| | pH 5.5 | 6.0 | 6.5 | 7.6 |
| Stress (-) | 0.90 | 1.36 | 1.55 | 1.23 |
| | | | | |
| Stress (+) | 0.73 | 1.15 | 1.30 | 0.92 |
| | | | | |
| Activity (%) {Stress(+)/(-)} | 80.6 | 84.7 | 83.8 | 74.5 |
| Stress: 50°C/10 min. | | | | |

### Example 4:

### Stability test on an ALP-AFP compound when the pH of buffers containing a stabilizing agent was changed:

### 1. Preparation of an ALP-AFP compound

An ALP-AFP compound was prepared in a manner similar to that described under item A in Example 2.

### 2. Preparation of buffers

In 100 ml each of 5 different buffers including two inventive stabilizing buffers (K₂HPO₄/KH₂PO₄, HEPES/sodium phytate) and 3 different conventional buffers (MES, HEPES, TEA), the concentration being 100 mM throughout the five buffers, the following were added: sodium chloride (150 mM), BSA (1%), magnesium chloride (2 mM), zinc chloride (0.2 mM), and sodium azide (0.1%). Subsequently, 6N HCl was used to adjust the pH as follows:
K₂HPO₄/KH₂PO₄: 5.5, 6.0, 6.5, 7.0, 7.5, 8.0, 8.5
HEPES/sodium phytate: 7.0, 7.5, 8.0
MES: 5.5, 6.0, 6.5
HEPES: 7.0, 7.5, 8.0, 8.5, and
TEA: 7.6.

### 3. Assay method

Using the above-described buffers, 1 µg/ml of solutions were prepared from the ALP-AFP compound at a high concentration prepared in 1 above. Subsequently, a heat load test described under item C in Example 2 was carried out. Activities of the compound were measured.

The results are shown in Tables 4 and 5.

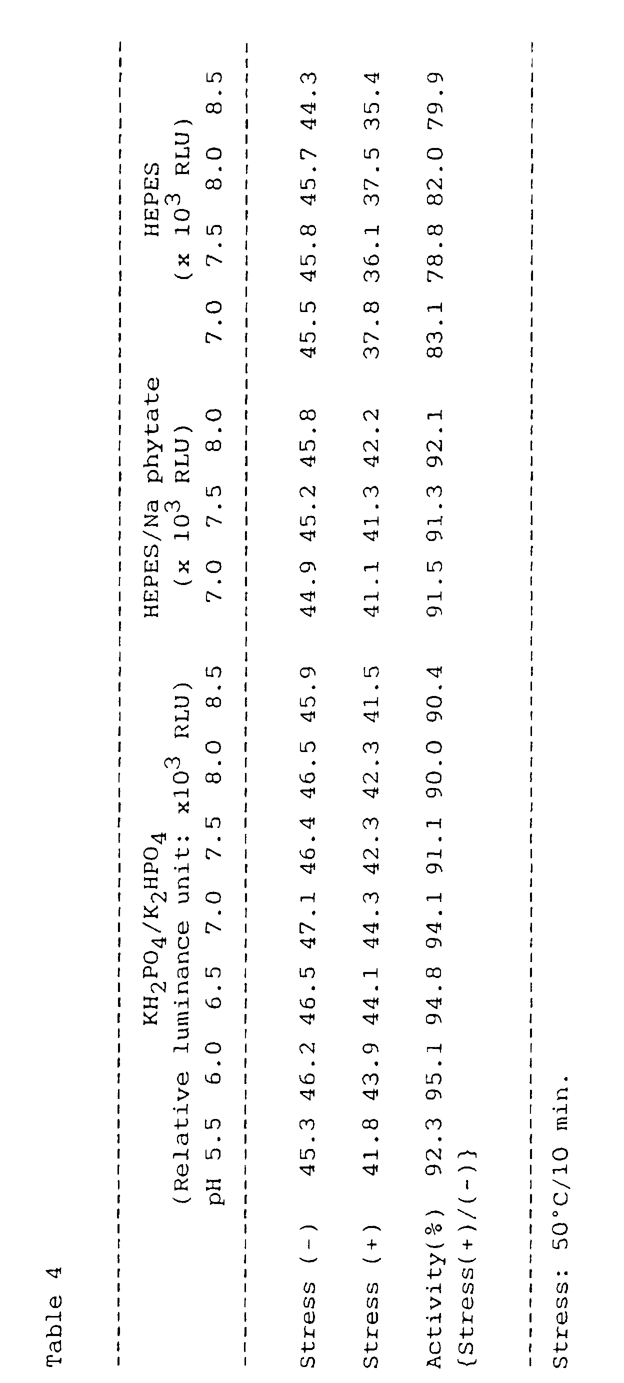

**Table 5**

| | MES (x 10³ RLU) | | | TEA (x 10³ RLU) |
|---|---|---|---|---|
| | pH 5.5 | 6.0 | 6.5 | 7.6 |
| Stress (-) | 44.5 | 45.8 | 44.1 | 42.8 |
| | | | | |
| Stress (+) | 37.1 | 38.1 | 36.9 | 33.2 |
| | | | | |
| Activity (%) {Stress(+)/(-)} | 83.3 | 83.1 | 83.6 | 77.5 |
| Stress: 50°C/10 min. | | | | |

As is apparent from Tables 4 and 5, in the two buffers containing stabilizing agents, stability of the enzyme or enzyme-labelled antibody was enhanced at any pH between 5.5 to 8.5 inclusive. The effects were prominent in the pH range from 5.5 to 7.5.

By contrast, in the buffers which did not contain stabilizing agents, improvement in stability was not observed at any pH.

### Example 5:

### Effects of varying concentrations of the stabilizing agents, sodium phytate and a phosphate on an ALP-AFP compound:

### 1. Preparation of an ALP-AFP compound

An ALP-AFP compound was prepared in a manner similar to that described under item A in Example 2.

### 2. Preparation of buffers

Three different buffers shown in Table 4: 1) a Tris buffer made of 130 mM Tris, 150 mM NaCl, 1% BSA, 2mM MgCl, 0.2 mM zinc chloride, 0.1% sodium azide. 2) Tris/sodium phytate buffers obtained by adding, to a Tris buffer, a stabilizing agent sodium phytate in amounts of 1, 2.5, 5.0, 10.0, or 40.0 mM. 3) KH₂PO₄/K₂HPO₄ buffers containing 10 mM to 250 mM KH₂PO₄/K₂HPO₄, 150 mM of NaCl, 1% BSA, 2 mM MgCl, 0.2 mM zinc chloride, 0.1% sodium azide. All of them were adjusted to have a pH 7.4.

### 3. Assay method

Using the above-described 11 different buffers, the ALP-AFP compound at a high concentration was diluted to prepare compound solutions each having a concentration of 1 µg/ml of ALP-AFP. The compound was subjected to a heat load test (37°C, 2 weeks). Control groups were stored at -80°C. After completion of the heat load test, residual activities were measured as in Example 2.

The results are shown in Table 6.

**Table 6**

| Buffer | Concentration (mM) | Stress(-) (RLU) | Stress(+) (RLU) | Activity(%) {Stress(+)/(-)} |
|---|---|---|---|---|
| Tris/Na phytate (pH 7.4) | 1.0 | 52,689 | 45,366 | 88.1 |
| | 2.5 | 51,965 | 46,778 | 90.5 |
| | 5.0 | 51,883 | 48,251 | 93.0 |
| | 10.0 | 52,317 | 48,951 | 93.5 |
| | 40.0 | 51,473 | 48,018 | 93.2 |
| | | | | |
| KH₂PO₄/K₂HPO₄ (pH 7.4) | 10.0 | 55,826 | 43,869 | 78.6 |
| | 20.0 | 56,264 | 47,824 | 84.6 |
| | 50.0 | 55,623 | 51,287 | 94.0 |
| | 100.0 | 54,288 | 51,053 | 94.0 |
| | 250.0 | 54,529 | 51,118 | 93.7 |
| | | | | |
| Tris (pH 7.4) (as a control) | 50.0 | 53,462 | 32,728 | 61.2 |

Both the sodium phytate and phosphate were found effective at all the pH values tested.

### Example 6:

### Effects of stabilizing agents on the stability of an ALP-AFP compound when left at room temperature:

### 1. Preparation of an ALP-AFP compound

An ALP-AFP compound was prepared in a manner similar to that described under item A in Example 2.

### 2. Preparation of buffers

Three different buffers were prepared: 130 mM Tris buffers (150 mM Tris, 1% BSA, 2mM MgCl, 0.2 mM zinc chloride, 0.1% sodium azide) combined with stabilizers (10.0 mM of sodium phytate, KH₂PO₄/K₂HPO₄), and combined with no stabilizers (control). All of them were adjusted to have a pH 7.0.

### 3. Assay method

Using the above-described 3 different buffers, the ALP-AFP compound of a high concentration prepared in item 1 above was diluted to prepare compound solutions each having a concentration of 1 µg/ml of ALP-AFP. The three compound solutions were allowed to stand at room temperature (25°C). The solutions were sampled every week starting from week 0, and residual activities were measured in a manner similar to that described under item C in Example 2.

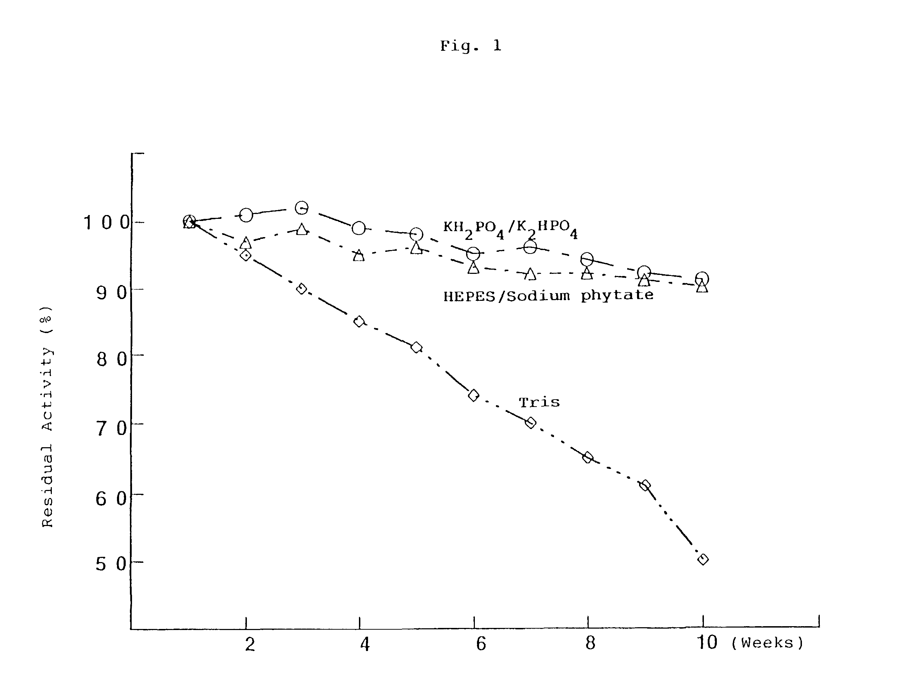

The results were shown in Fig. 1.

As is apparent from Fig. 1, use of sodium phytate or KH₂PO₄ improved stability at room temperature.

### Example 7:

### Effects of stabilizers on the stability of an ALP-AFP compound at various temperatures:

### 1. Preparation of an ALP-AFP compound

An ALP-AFP compound was prepared in a manner similar to that described under item A in Example 2.

### 2. Preparation of buffers

Buffers were prepared in a manner similar to that described under item 2 in Example 6.

### 3. Assay method

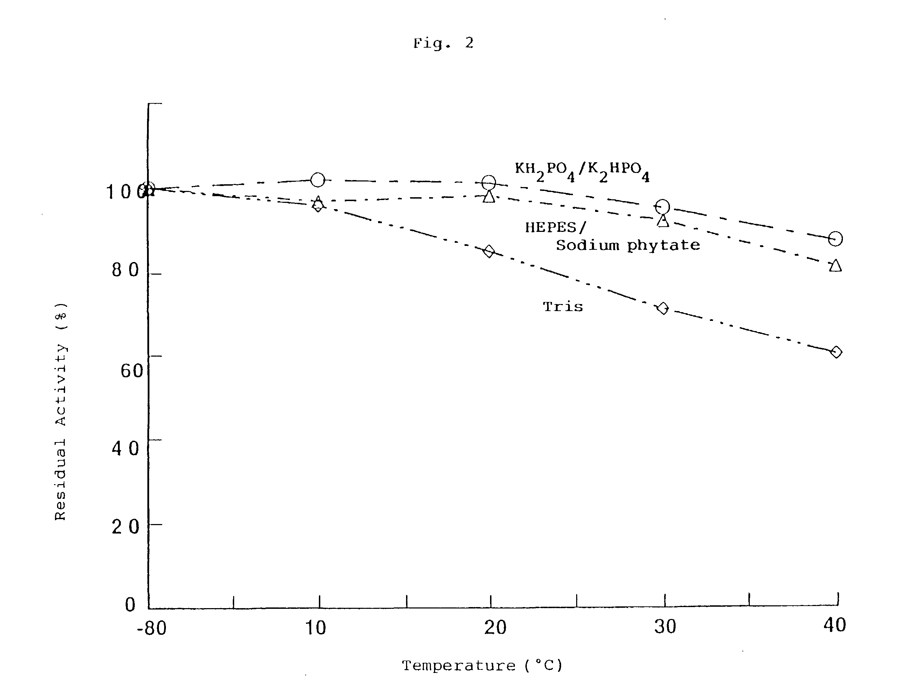

Using the above-described 3 different buffers, the ALP-AFP compound of a high concentration prepared in item 1 above was diluted to prepare compound solutions each having a concentration of 1 µg/ml of ALP-AFP. The solutions were allowed to stand for 10 minutes at 10°C, 20°C, 25°C, 30°C, and 40°C. 50 µl of each solution was sampled and residual activities were measured in a manner similar to that described under Item C in Example 1. The results are shown in Fig. 2.

As is apparent from Fig. 2, after the stabilizing agents of the present invention were added, resistance against heat was improved compared to cases where the stabilizing agents were not added to buffers containing the enzyme.

## Claims

1. An enzymatic solution comprising alkaline phosphatase or a derivative thereof and a compound having a binding activity reversible to the alkaline phosphatase,
wherein said compound is selected from the group consisting of a phosphoric acid ester, an alkali, metal salt of phosphoric acid or a phosphoric acid ester, and a mixture of them.

2. The enzymatic solution according to Claim 1, wherein the alkaline phosphatase or derivative thereof is alkaline phosphatase or a compound of alkaline phosphatase and an antigen, antibody, avidin, peptide, hapten, coenzyme, sugar or a nucleic acid.

## Patentansprüche

1. Enzymatische Lösung umfassend alkalische Phosphatase oder ein Derivat davon und eine Verbindung, die gegenüber der alkalischen Phosphatase eine reversible Bindungsaktivität aufweist, wobei die Verbindung ausgewählt ist aus der Gruppe bestehend aus einem Phosphorsäureester, einem Alkalimetallsalz von Phosphorsäure oder einem Phosphorsäureester und einem Gemisch davon.

2. Enzymatische Lösung nach Anspruch 1, worin die alkalische Phosphatase oder das Derivat davon eine alkalische Phosphatase oder eine Verbindung von alkalischer Phosphatase und einem Antigen, Antikörper, Avidin, Peptid, Hapten, Coenzym, Zucker oder einer Nukleinsäure ist.

## Revendications

1. Solution enzymatique comprenant une phosphatase alcaline ou un dérivé de celle-ci et un composé ayant une activité de liaison réversible vis-à-vis de la phosphatase alcaline, dans laquelle ledit composé est choisi dans le groupe consistant en un ester d'acide phosphorique, un sel de métal alcalin d'acide phosphorique ou un ester d'acide phosphorique, et un mélange de ceux-ci.

2. Solution enzymatique selon la revendication 1, dans laquelle la phosphatase alcaline ou un dérivé de celle-ci est une phosphatase alcaline ou un composé d'une phosphatase alcaline et un antigène, un anticorps, de l'avidine, un peptide, un haptène, une coenzyme, un sucre ou un acide nucléique.
